# EUROPÄISCHE PATENTSCHRIFT

(11) **EP 0 620 041 B1**
(45) Veröffentlichungstag und Bekanntmachung des Hinweises auf die Patenterteilung: **03.09.1997**
(21) Anmeldenummer: 94105137.7
(22) Anmeldetag: 31.03.1994
(51) Int. Cl.: B01J 31/10, C07C 37/20

(54) **Mehrfach regenerierbare Ionenaustauscherharze mit geringer Alkyl-SH-Gruppen-Belegung**
Ion exchange resin for multiple regeneration with a low alkylmercaptan group content
Résine échangeuse d'ions pour régénérations multiples à faible teneur en groupes alkylmercaptans

(30) Priorität: 13.04.1993 DE 4312038
(43) Veröffentlichungstag der Anmeldung: 19.10.1994
(73) Patentinhaber: BAYER AG, 51368 Leverkusen (DE)
(72) Erfinder: Berg, Klaus, Dr., D-47798 Krefeld (DE); Malamet, Georg, Dr., D-47800 Krefeld (DE); Backes, Franz, Dr., D-47829 Krefeld (DE); Eitel, Alfred, Dr., D-41539 Dormagen (DE); Wulff, Claus, Dr., D-47800 Krefeld (DE)

(56) Entgegenhaltungen:
- EP-A- 0 023 325
- EP-A- 0 049 411
- EP-A- 0 268 318

## Beschreibung

Die Erfindung betrifft mehrach regenerierbare Ionenaustauscherharze mit geringer Alkyl-SH-Gruppen-Belegung.

Die Kondensation von Phenolen und Carbonylverbindungen zur Bildung von Bisphenolen ist bekannt. Bei dieser Reaktion wurden bereits die verschiedensten Katalysatoren verwendet, beispielsweise Chlorwasserstoffsäure (US-PS 21 82 308 und 21 91 831), Bortrifluorid (Chemical Abstracts 58, 3338 c), Perchlorsäure (Chemical Abstracts 60, 1626 h), Benzolsulfonsäure (Chemical Abstracts 59, 511 h) und die verschiedensten Kationenaustauschharze (z.B. GB-PS 8 42 209, 8 49 565 und 8 83 391). Auch der Zusatz von S-haltigen Verbindungen zum Katalysator ist bekannt, z.B. aus der US-PS 24 68 982, 26 23 908 die Verwendung von Thioglykolsäure-und 3-Mercaptopropionsäure, aus der US-PS 23 59 242 der Zusatz von Thiophenolen, aus der US-PS 27 75 620 der Zusatz von Alkylmercaptanen, aus Chemical Abstracts 58, 1403 e, der Zusatz von Schwefelwasserstoff.

Die US-PS 33 94 089 beschreibt ein Verfahren zur Herstellung von Bisphenol aus Aceton und Phenol unter Verwendung eines sulfonsäuregruppenhaltigen Katalysators, dessen Sulfonsäuregruppen zu 5 bis 25 Mol-% mit Mercaptoaminen belegt worden sind. Durch die Fixierung cokatalytisch aktiver Verbindungen an der Harzmatix wird
a) die Selektivität und Reaktivität der Bisphenol-Synthese verbessert und b) die Verunreinigung des Endprodukts mit Schwefelverbindungen vermieden.

Ans der DE-A 3619 450 ist ein Verfahren bekannt in dem die Belegung des Ionenaustanschers mit Mercaptoalkylaminen in phenolfeuchter Phase durchgeführt wird Hierdurch soll eine besondere Langlebigkeit des Harzes erzielt werden. Das Harz hat eine Sulfonsäuregruppen-Belegung von 30 bis 100 Mol-% (bezogen auf Sulfonsäuregruppen).

Es hat sich aber gezeigt, daß die Standzeit mit SH-Gruppen modifizierter Ionenanstauschersysteme im Vergleich zu nichtmodifizierten um den Faktor 10 sinkt. Hierdurch werden die Vorteile des modifizierten Systems aufgehoben, da derartige Standzeiten wirschaftlich nicht vertretbar sind

Sind beim Ionenaustauschersystem bei der Erstbeleung 5 Mol-% und mehr der -SO₃H-Gruppen belegt worden, wird nach Desaktivierung und erneuter Belegung nicht mehr die ursprüngliche Umsatzaktivität erreicht. Eine dritte Nachbelegung nach erneuter Belegung erreicht nicht mehr die ursprüngliche Umsatzaktivität und ist wegen der dann stark geminderten Reaktivität des Ionenaustauschers nicht mehr Iohnenswert. Der Ionenaustauscher muß folglich anschließend in aufwendiger Weise regeneriert oder unter hohen Kosten entsorgt werden. Es resultieren also sehr geringe Standzeiten.

Es wurde nun gefunden, daß ein Ionenaustauscher mit einer Belegung <5 Mol-% nach seiner Desaktivierung dadurch wieder regeneriert werden kann, daß erneut eine Belegung mit Alkyl-SH-Gruppen enthaltenden Spezies vorgenommen wird

Gegenstand der Erfindung sind somit makroporöse oder gelförmige sulfonsaure Ionenaustauscherharze mit Vernetzungsgraden von 1 bis 20 % deren Sulfonsäuregruppen zu 1 bis 3 Mol-% mit Alkyl-SH-Gruppen enthaltenden Spezies belegt worden sind

Diese Belegeng kann durch eine ionische oder kovalente Bindung erfolgen. Als Beispiel für die Spezies seien hier Mercaptoethylamine oder deren Vorläufer (Thiazolidine), wie in der US-PS 33 94 089 und der DE-A 36 19 450 beschrieben, genannt. Als Matrix können gelartige oder makroporöse, sulfonsaure Ionenaustauscher mit Vernetzungsgraden von 1 bis 20 % benutzt werden. Die Aufbringung der ionisch fixierten Einheiten kann nach Verfahren erfolgen, wie sie in der US-PS 33 94 089 oder der DE-A 36 19 450 beschrieben worden sind.

Der erfindungsgemäße Belegungsgrad des Ionenaustauschers mit Alkyl-SH-Einheiten beträgt 1 bis 3 Mol-% und zeigt folgende Vorteile:
a) die Reaktivität und Selektivität derartiger Katalysatorsysteme ist nur unwesentlich geringer als die der höher belegten;
b) die Standzeit eines mit 1 bis 3 Mol-% Alkyl-SH-Einheiten bezogen auf SO₃H-Gruppen belegten Katalysatorharzes ist der eines höherbelegten (≥5 Mol-%) vergleichbar;
c) das gering belegte Harz ist mehrfach (bis zu 5 mal) durch Nachbelegung mit 1 bis 3 Mol-% SH-Alkyl-Einheiten (bezogen auf SO₃H-Gruppen) regenerierbar, ohne daß seine Aktivität gravierend abfällt. Dadurch erhöh sich seine Standzeit
gegenüber den höherbelegten Systemen um den Faktor 3 bis 5.

Als Alkyl-SH-Gruppen enthaltende Spezies werden erfindungsgemäß Mercaptoethylamine der Formel (I)

HS-CH₂CH₂-NR^{¹}R² (I),

in welcher
R^{¹} und R² unabhängig voneinander für Wasserstoff (H) oder C₁-C₄-Alkyl, stehen,
oder Thiazolidine der Formel (II) 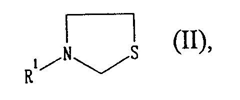 in welcher
R¹ für Wasserstoff (H) oder C₁-C₄-Alkyl steht,
eingesetzt.

Es können Gemische der Verbindungen der Formeln (I) und (II) eingesetzt werden.

Die Herstellung z.B. von Bisphenol-A mit dem erfindungsgemäß belegtem Ionenaustauscher kann konti- oder diskontinuierlich durchgeführt werden Die Reaktionstemperatur bei der Herstellung der Bisphenole liegt im Bereich von 40 bis 120°C, vorzugsweise oberhalb des Erstarrungspunktes der beteiligten Komponenten. Das nach der Umsetzung von Phenol- und Carbonylverbindung erhaltene Reaktionsgemisch wird nach üblichen, bekannten Methoden wie Kristallisation, Destillation etc. aufgearbeitet.

Die erfindungsgemäßen Ionenaustauscher können hergestellt werden durch Belegung von sulfonierten Polystyrolharzen mit Mercaptoalkylaminen der Formel (I) und/oder Thiazolidinen der Formel (II).

Weiterhin können desaktivierte Ionenaustauscher, deren Sulfonsäuregruppen bereits ein- oder mehrmals zu 1 bis 3 Mol-% mit Alkyl-SH-Einheiten belegt worden waren, durch erneute Nachbelegung mit Alkyl-SH-Einheiten von 1 bis 3 Mol-% (bezogen auf die Sulfonsäuregruppen) regeneriert werden. Es können auch, gegebenenfalls mit vermindertem Wirkungsgrad, Harze wiederbelegt werden, die ursprünglich bis zu 25 Mol-% (bezogen auf Sulfonsäureeinheiten) Alkyl-SH-Einheiten enthielten.

### Beispiele

### Beispiel 1

Herstellung des modifizierten Ionenaustauscherharzes (handelsübliches Polystyrol, sulfoniert, mit einem Vernetzungsgrad von 1-20 %):

Der wasserfeuchte Ionenaustauscher (ca 80 Gew-% Wasser-Feuchte) mit einer Totalkapazität von 0,75 mval/ml in Lieferform wird zunächst mit destilliertem Wasser gewaschen. Anschließend wird das Harz bei 90 bis 100°C und Wasserstrahlpumpenvakuum 24 Stunden lang getrockent, so daß der Wassergehalt auf <1 Gew.-% fällt.

Das Restwasser wird im azeotropen Gemisch mit Toluol abdestilliert und danach das dem Ionenaustauscherharz noch anhaftende Toluol bei 95°C im Wasserstrahlpumpanvakuum abdestilliert.

120 g des so vorbehandelten Ionenaustuascherharzes werden in einer Rührapparatur in 1,128 g Phenol aufgenommen und 24 h unter Feuchtigkeitsausschluß bei 65°C gequollen. Danach wird unter Rühren die für eine bestimmte Belegung in Mol-% der Beispiele 2 bis 10 erforderliche Menge Mercaptoethylamin zugegeben.

### Beispiel 2

Mit dem unter Beispiel 1 beschriebenen Verfahren wurde ein Ionenaustauscherharz präpariert, dessen Sulfonsäuregruppen zu 5 Mol-% mit Cysteamin (NH₂-CH₂-CH₂-SH) belegt wurden. Mit dem Harz wurde ein Standardansatz durchgeführt:

Zu 250 g phenolfeuchtem Ionenaustauscher wurden 1,128 g Phenol und 58 g Aceton gegeben und in einer Rührapparatur 4 Stunden bei 65°C gerührt. Danach wird gaschromatographisch der Umsatz und die Selektivität der Reaktion bestimmt (Standardansatz).
- Umsatz:: 95,5 %
- Selektivität:: 94,0 %

### Beispiel 3

Der im Beispiel 2 beschriebene Versuch wird mit einem Harz wiederholt, dessen Sulfonsäuregruppen zu 2,8 Mol-% mit Mercaptoethylamin belegt worden sind
- Umsatz:: 94,9 %
- Selektivität:: 93,8 %

### Beispiel 4

Ein ursprünglich mit 5 Mol-% Mercaptoethylamin belegter, inzwischen desaktivierter Ionenaustauscher wurde mit 5 Mol-% Mercaptoethylamin nachbelegt und mit ihm wie im Beispiel 2 ein Standardansatz gefahren:
- Umsatz:: 75,3 %
- Selektivität:: 93,7 %

### Beispiel 5

Ein mit ursprünglich 2,8 Mol-% Mercaptoethylamin belegter, desaktivierter, danach mit 2,8 Mol-% erneut nachbelegter und wiederum desaktivierter Ionenaustauscher wurde mit 2,8 Mol-% Mercaptoethylamin erneut nachbelegt und ein Standardansatz gefahren:
- Umsatz:: 90,1 %
- Selektivität:: 93,1 %

### Beispiel 6 (Vergleich)

Ein mit ursprünglich 5 Mol-% Mercaptoethylamin belegter, desaktivierter, danach mit 5 Mol-% Mercaptoethylamin erneut nachbelegter und wiederum desaktivierter Ionenaustauscher wurde mit 5 Mol-% Mercaptoethylamin erneut nachbelegt und ein Standardansatz gefahren:
- Umsatz:: 48,7 %
- Selektivität:: 90,4 %

### Beispiel 7 (Vergleich)

Ein ursprünglich mit 5 Mol-% Mercaptoethylamin belegter, inzwischen desaktivierter Ionenaustauscher wurde mittels Standardansatz gemäß Beispiel 1 überprüft.
- Umsatz:: 29,2 %
- Selektivität:: 80,3 %

## Patentansprüche

1. Sulfonsaure Ionenaustauscher, makroporös oder vom gelförmigen Typ, mit Vernetzungsgraden von 1 bis 20 %, dadurch gekennzeichnet, daß sie von 1 bis 3 Mol-%, bezogen auf Sulfonsäuregruppen, Alkyl-SH-Einheiten, ionisch oder kovalent fixiert, enthalten.

## Claims

1. Sulphonic acid ion exchangers which are macroporous or of the gel-form type, having degrees of crosslinking from 1 to 20 %, characterised in that they contain from 1 to 3 mole %, with respect to sulphonic acid groups, of alkyl-SH units fixed ionically or covalently.

## Revendications

1. Echangeurs d'ions sulfonés, macroporeux ou du type en forme de gel, possédant des degrés de réticulation de 1 à 20%, caractérisés en ce qu'ils contiennent, à concurrence de 1 à 3 moles % rapportés aux groupes d'acides sulfoniques, des unités alkylmercaptans fixées par liaison ionique ou covalente.
